# EUROPEAN PATENT APPLICATION

(11) **EP 4 477 729 A1**
(43) Date of publication of application: **18.12.2024**
(21) Application number: 23752603.3
(22) Date of filing: 13.01.2023
(51) Int. Cl.: C10G 2/00, B01D 53/22, B01J 8/02, C07C 29/152, C07C 31/04

(54) **REACTOR**

(30) Priority: 08.02.2022 JP 2022017954
(71) Applicant: NGK Insulators, Ltd., Nagoya-shi, Aichi 467-8530 (JP)
(72) Inventor: NAKAGAWA, Kosuke, Nagoya-shi, Aichi 467-8530 (JP); IIDA, Kazuki, Nagoya-shi, Aichi 467-8530 (JP); KAN, Hirofumi, Nagoya-shi, Aichi 467-8530 (JP); TORII, Atsushi, Nagoya-shi, Aichi 467-8530 (JP)
(74) Representative: Müller-Boré & Partner Patentanwälte PartG mbB
(86) International application number: PCT/JP2023/000763
(87) International publication number: WO 2023/153134

(57) **Abstract**

A reactor (1) is provided with a separation membrane (10) permeable to water vapor, a non-permeation side flow path (30) provided on a non-permeation side of the separation membrane (10), and a catalyst (60) provided in ling the non-permeable side flow path (30). The catalyst (60) includes catalyst particles each constituted by a carrier and a supported catalytic component, and filler particles softer than the catalyst particles.

## Description

### TECHNICAL FIELD

The present invention relates to a reactor.

### BACKGROUND ART

In recent years, reactors have been developed that can improve conversion efficiency by separating a product of a conversion reaction in which a raw material gas containing hydrogen and carbon oxide is converted to a liquid fuel such as methanol and ethanol (specifically, a fuel that is in a liquid state at normal temperature and pressure).

For example, Patent Literature 1 discloses a reactor provided with a separation membrane permeable to water vapor that is one of products of the conversion reaction, a non-permeation side flow path through which a raw material gas flows, and a catalyst filling the flow path. The catalyst promotes the conversion reaction in which the raw material gas is converted to the liquid fuel.

### CITATION LIST

### Patent Literature

Patent Literature 1: JP 2018-8940A

### SUMMARY

### TECHNICAL PROBLEM

In the reactor disclosed in Patent Literature 1, when a high-temperature material gas flows into the non-permeation side flow path at the start of operation, the catalyst thermally expands and the separation membrane may be damaged, and in addition, the catalyst itself may be pulverized due to thermal stress generated in the catalyst.

An object of the present invention is to provide a reactor capable of both suppressing damage to a separation membrane and suppressing pulverization of the catalyst.

### SOLUTION TO PROBLEM

A reactor according to a first aspect of the present invention is a reactor including a separation membrane permeable to a product of a conversion reaction in which a raw material gas containing at least hydrogen and carbon oxide is converted to a liquid fuel, a non-permeation side flow path provided on a non-permeation side of the separation membrane, the raw material gas flowing through the non-permeation side flow path, and a catalyst provided in the non-permeation side flow path and configured to promote the conversion reaction. The catalyst includes catalyst particles each constituted by a carrier and a supported catalytic component, and filler particles softer than the catalyst particles.

A reactor according to a second aspect of the present invention is the reactor according to the first aspect, in which the non-permeation side flow path extends in an approximately vertical direction, the catalyst has a first layer and a second layer disposed higher than the first layer, the first layer includes more of the filler particles than the catalyst particles, and the second layer includes more of the catalyst particles than the filler particles.

A reactor according to a third aspect of the present invention is the reactor according to the first or second aspect, in which the non-permeation side flow path extends in an approximately vertical direction, the catalyst has a first layer and a second layer disposed higher than the first layer, the filler particles include first filler particles disposed in the first layer and second filler particles disposed in the second layer, and the first filler particles are softer than the second filler particles.

A reactor according to a fourth aspect of the present invention is the reactor according to the third aspect, in which a porosity of the first layer is larger than a porosity of the second layer.

A reactor according to a fifth aspect of the present invention is the reactor according to the second aspect, in which the first layer is located at a lowermost layer of the catalyst.

A reactor according to a sixth aspect of the present invention is the reactor according to the first aspect, in which the catalyst has a first portion in contact with the separation membrane, and a second portion separated from the separation membrane, the first portion includes more of the filler particles than the catalyst particles, and the second portion includes more of the catalyst particles than the filler particles.

A reactor according to a seventh aspect of the present invention is the reactor according to the first or sixth aspect, in which the catalyst has a first portion in contact with the separation membrane, and a second portion separated from the separation membrane, the filler particles include third filler particles disposed in the first portion and fourth filler particles disposed in the second portion, and the third filler particles are softer than the fourth filler particles.

A reactor according to an eighth aspect of the present invention is the reactor according to the sixth aspect, in which a porosity of the first portion is larger than a porosity of the second portion.

A reactor according to a ninth aspect of the present invention is the reactor according to the fist aspect, in which the catalyst has a first portion in contact with the separation membrane, and a second portion separated from the separation membrane, and the first portion includes more of the catalyst particles than the filler particles, and the second portion includes more of the filler particles than the catalyst particles.

A reactor according to a tenth aspect of the present invention is the reactor according to the first or ninth aspect, in which the catalyst has a first portion in contact with the separation membrane, and a second portion spaced apart from the separation membrane, the filler particles include fifth filler particles disposed in the first portion and sixth filler particles disposed in the second portion, and the sixth filler particles are softer than the fifth filler particles.

A reactor according to an eleventh aspect of the present invention is the reactor according to the ninth aspect, in which a porosity of the second portion is larger than a porosity of the third portion.

A reactor according to a twelfth aspect of the present invention is the reactor according to the first aspect, in which the filler particles are each constituted by a carrier and a supported catalytic component.

### ADVANTAGEOUS EFFECTS

According to the present invention, a reactor capable of both suppressing damage to a separation membrane and suppressing pulverization of a catalyst can be provided.

### BRIEF DESCRIPTION OF DRAWINGS

FIG. 1 is a cross-sectional view of a reactor according to a first embodiment.
FIG. 2 is a cross-sectional view of a reactor according to a second embodiment.
FIG. 3 is a cross-sectional view of a reactor according to a second comparative example.
FIG. 4 is a cross-sectional view of the reactor according to the second comparative example.
FIG. 5 is a cross-sectional view of a reactor according to a third comparative example.

### DESCRIPTION OF EMBODIMENTS

Embodiments of the present invention will be described with reference to the drawings. However, the drawings are schematic, and the ratio or the like of dimensions may differ from an actual one.

### 1. First Embodiment

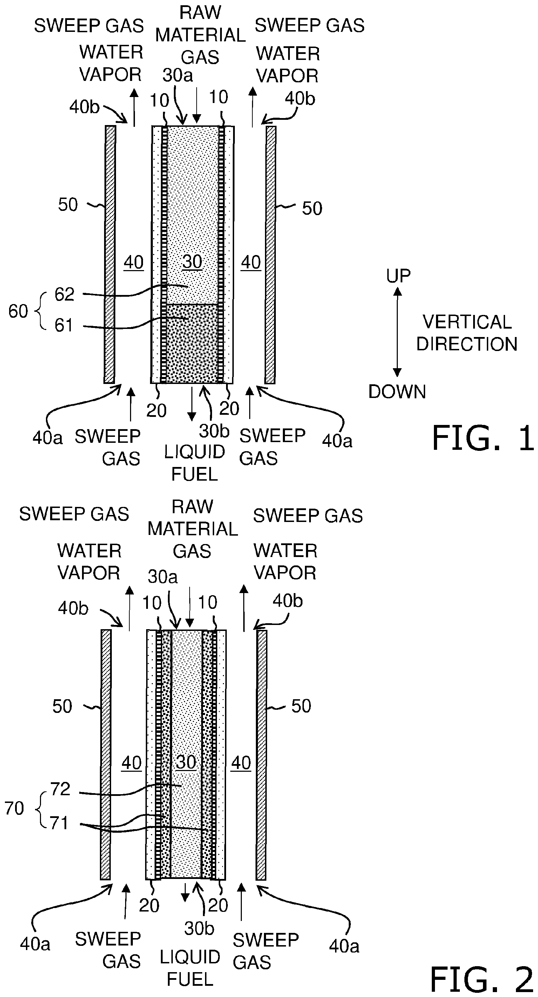

FIG. 1 is a cross-sectional view of a reactor 1 according to a first embodiment.

The reactor 1 is a so-called membrane reactor used to convert a raw material gas to a liquid fuel. The configuration of the reactor 1 can be applied to a fixed bed reactor and a monolith-type reactor.

The raw material gas contains at least hydrogen and carbon oxide. At least one of carbon monoxide and carbon dioxide can be used as the carbon oxide. The raw material gas may be a so-called synthetic gas (syngas).

The liquid fuel is a fuel that is in a liquid state at normal temperature and pressure, or a fuel that can be liquidized at normal temperature and pressurized state. Examples of the liquid fuel that is in a liquid state at normal temperature and pressure may include methanol, ethanol, liquid fuels represented by CₙH₂₍ₘ₋₂ₙ₎ (m is an integer less than 90, and n is an integer less than 30), and mixtures thereof. Examples of the fuel that can be liquidized at normal temperature and pressurized state include, for example, propane, butane, and mixtures thereof.

For example, reaction formula (1) for synthesizing methanol by catalytically hydrogenating a raw material gas containing carbon dioxide and hydrogen in the presence of a catalyst is as follows.

CO₂ + 3H₂ ↔ CH₃OH + H₂O (1)

The above reaction is an equilibrium reaction, and in order to increase both the conversion efficiency and the reaction rate, it is preferable to carry out the reactions at a high temperature and high pressure (for example, 180°C or higher and 2 MPa or higher). The liquid fuel is in a gaseous state when it is synthesized, and is kept in a gaseous state at least until it flows out of the reactor 1. The reactor 1 preferably has heat resistance and pressure resistance suitable for desired synthesis conditions of the liquid fuel.

As shown in FIG. 1, the reactor 1 includes a separation membrane 10, a porous support body 20, a non-permeation side flow path 30, a permeation side flow path 40, an outer tube 50, and a catalyst 60.

The separation membrane 10 is permeable to water vapor, which is one of the products of the conversion reaction of the raw material gas to the liquid fuel. In this manner, by utilizing the equilibrium shift effect, the reaction equilibrium of the above formula (1) can be shifted to the product side. In the present embodiment, the separation membrane 10 is formed in a cylindrical shape.

The separation membrane 10 preferably has a water vapor permeability coefficient of 100 nmol/(s·Pa·m²) or more. The water vapor permeability coefficient can be determined using a known method (see Ind. Eng. Chem. Res., 40, 163-175 (2001)).

The separation membrane 10 is formed in a tubular shape. The separation membrane 10 is disposed inside a cylindrical porous support body 20. The separation membrane 10 is in contact with the inner peripheral surface of the porous support body 20.

The separation membrane 10 preferably has a separation factor of 100 or more. The greater the separation factor is, the more permeable the separation membrane 10 is to water vapor, and the less permeable it is to components other than water vapor (e.g., hydrogen, carbon oxide, and liquid fuel). The separation factor can be determined using a known method (see FIG.1 of "Separation and Purification Technology 239 (2020) 116533").

An inorganic membrane can be used as the separation membrane 10. The inorganic membrane is preferable because it has heat resistance, pressure resistance, and water vapor resistance. Examples of the inorganic membrane include a zeolite membrane, a silica membrane, an alumina membrane, and a composite membrane thereof. In particular, an LTA zeolite membrane having a molar ratio (Si/Al) of a silicon element (Si) and an aluminum element (Al) of 1.0 or more and 3.0 or less is suitable because of its excellent water vapor permeability. Note that the inorganic membrane has a characteristic of being easily broken by thermal shock.

The porous support body 20 supports the separation membrane 10. The porous support body 20 is constituted by a porous material. In the present embodiment, the porous support body 20 is formed in a cylindrical shape.

As the porous material, a ceramic material, a metal material, a resin material, or the like can be used, and a ceramic material is particularly preferable. As an aggregate of the ceramic material, for example, at least one of alumina (Al₂O₃), titania (TiO₂), mullite (Al₂O₃-SiO₂), potsherd, cordierite (Mg₂Al₄Si₅O₁₈) can be used. As an inorganic binder for the ceramic material, for example, at least one of titania, mullite, readily sinterable alumina, silica, glass frit, a clay mineral, and readily sinterable cordierite can be used. Note that the ceramic material does not need to contain an inorganic binder.

The porous support body 20 is formed in a tubular shape. The porous support body 20 surrounds the separation membrane 10. The non-permeation side flow path 30 is provided on a non-permeation side of the separation membrane 10. In the present embodiment, the non-permeation side flow path 30 is a columnar space on the inner side of the separation membrane 10.

In the present embodiment, the non-permeation side flow path 30 extends in an approximately vertical direction. The approximately vertical direction is a concept including not only a direction that matches the gravity direction, but also a direction inclined to some extent (±15°) with respect to the gravity direction.

The raw material gas is caused to flow through the non-permeation side flow path 30. In the present embodiment, the raw material gas is caused to flow downward through the non-permeation side flow path 30. Accordingly, the raw material gas flows in through an upper end opening 30a of the non-permeation side flow path 30, and the liquid fuel flows out through a lower end opening 30b of the non-permeation side flow path 30. Note that the raw material gas may be caused to flow upward through the non-permeation side flow path 30. The liquid fuel flowing out through the lower end opening 30b may be mixed with a residual raw material gas.

The permeation side flow path 40 is provided on the non-permeation side of the separation membrane 10. In the present embodiment, the permeation side flow path 40 is an annular space between the separation membrane 10 and the outer tube 50. Water vapor that has permeated through the separation membrane 10 flows into the permeation side flow path 40.

In the present embodiment, a sweep gas for sweeping water vapor is caused to flow through the permeation side flow path 40. An inert gas (e.g., nitrogen), air, and the like can be used as the sweep gas. In the present embodiment, the sweep gas flows upward (i.e., the opposite direction to the direction in which the raw material gas flows) through the permeation side flow path 40. Accordingly, the sweep gas flows in through the lower end opening 40a of the permeation side flow path 40, and the sweep gas that has taken in water vapor flows out through the upper end opening 40b of the permeation side flow path 40. Note that the sweep gas may be caused to flow downward (i.e., the same direction as the direction in which the raw material gas flows) through the permeation side flow path 40.

The catalyst 60 fills the non-permeation side flow path 30. The catalyst 60 advances (promotes) the conversion reaction of the raw material gas to the liquid fuel. The catalyst 60 is in direct contact with the separation membrane 10.

The catalyst 60 is constituted by a plurality of catalyst particles and a plurality of filler particles.

The catalyst particles are each constituted by a carrier and a supported catalytic component that exhibits catalysis. As the carrier, for example, alumina, titania, silica, ceria, zeolite or the like can be used, but there is no limitation to this. The supported catalytic component is supported on a surface of the carrier. As the supported catalytic component, metal catalyst component (copper, palladium, and the like), oxide catalyst component (zinc oxide, amorphous zirconia, gallium oxide, and the like), and composite catalyst components thereof can be used, but there is no limitation to this.

The shape of the catalyst particles is not particularly limited, and for example, may be a spherical shape, an ellipsoidal shape, a circular columnar shape, an ellipsoidal columnar shape, a disc-like shape, a scale-like shape, a needle-like shape, a polygonal columnar shape, a sheet-like shape, or the like.

The filler particles are constituted of a material that is softer than the catalyst particles. Examples of the material that is softer than the catalyst particles include alumina, titania, silica, ceria, and zeolite, but there is no limitation to this.

The filler particles may be constituted by a carrier and a supported catalytic component that exhibits catalysis. In this manner, the overall catalysis of the catalyst 60 can be improved. As the carrier, for example, alumina, titania, silica, ceria, zeolite or the like can be used, but there is no limitation to this. As the supported catalytic component, any of the above-described materials may be used, but there is no limitation to this.

In the present specification, "the filler particles are softer than the catalyst particles" means that the hardness of the filler particles is smaller than the hardness of the catalyst particles. The hardness of the filler particles and the hardness of the catalyst particles are measured by the Micro Autograph MST-I (manufactured by Shimadzu Corporation) using a sample that has been dried for two hours at 130 C°. The test speed is 0.10 mm/s.

The catalyst 60 has a multilayer structure in which a plurality of layers are stacked in the approximately vertical direction. In the present embodiment, the catalyst 60 is configured as a double-layer structure having a first layer 61 and a second layer 62.

The first layer 61 is located at the lowermost layer of the catalyst 60. The first layer 61 is disposed in the lower end portion of the non-permeation side flow path 30. The lower end portion of the non-permeation side flow path 30 is a region that is located at or lower than the center of the non-permeation side flow path 30 in the approximately vertical direction. The upper end of the first layer 61 may be located lower than the center of the non-permeation side flow path 30 in the approximately vertical direction.

The second layer 62 is disposed upward of the first layer 61. Since the catalyst 60 has the double layer structure in the present embodiment, the second layer 62 is disposed on the first layer 61. The second layer 62 is disposed in a region, of the non-permeation side flow path 30, where the first layer 61 is not disposed.

Here, the first layer 61 includes more of the filler particles than the catalyst particles. In other words, in the first layer 61, the content rate of the filler particles is larger than the content rate of the catalyst particles. Therefore, the first layer 61 is flexible. As such, when the reactor 1 starts operating, even if a high-temperature material gas flows through the non-permeation side flow path 30 and the first layer 61 thermally expands, the amount of force that the separation membrane 10 receives from the first layer 61 can be reduced. Further, since the weight of the second layer 62 that acts on the first layer 61 can be absorbed inside the first layer 61, the amount of force that the separation membrane 10 receives from the first layer 61 can be reduced. As a result, damage to the separation membrane 10 can be suppressed.

The second layer 62 includes more of the catalyst particles than the filler particles. In other words, in the second layer 62, the content rate of the catalyst particles is larger than the content rate of the filler particles. Since the catalyst particles are constituted of a material that is harder than the filler particles, pulverization of the catalyst particles due to thermal stress can be suppressed.

As described above, according to the catalyst 60 according to the present embodiment, both suppression of damage to the separation membrane 10 and suppression of pulverization of the catalyst 60 can be achieved.

The fact that the first layer 61 includes more of the filler particles than the catalyst particles can be confirmed as follows.

First, a resin (e.g., epoxy) is cast in the non-permeation side flow path 30 to be cured, and then the catalyst 60 is cut along the approximately vertical direction.

Next, a cross-sectional image of the first layer 61 is obtained using a SEM (Scanning Electron Microscope). The magnifying power of the SEM is selected from a range of 1-fold or more and 100-fold or less such that 10 or more (preferably 100 or more) filler particles can be observed in one observation field. In this case, when both cross sections of the separation membrane (cross sections on both sides in the horizontal direction of the non-permeation side flow path 30) are not seen in one observation field, the observation field can be divided into two observation fields for observing the cross sections of the separation membrane and one or more observation fields between the two observation fields. In the cross-sectional image, by classifying the brightness of the image into 256 tones, differences in brightness can be provided between the catalyst particles, the filler particles, and voids.

Next, total areas of the catalyst particles, the filler particles, and the voids were obtained by analyzing cross-sectional images using an image-analysis software HALCON (manufactured by MVTec AD (Germany)). If the total area of the filler particles is larger than the total area of the catalyst particles, it can be said that the first layer 61 contains more of the filler particles than the catalyst particles.

The content rate of the filler particles can be determined by dividing the total area of the filler particles by the sum of the total areas of the catalyst particles, the filler particles, and the voids. The content rate of the catalyst particles can be determined by dividing the total area of the catalyst particles by the sum of the total areas of the catalyst particles, the filler particles, and the voids.

Note that the first layer 61 need not substantially include the catalyst particles. Accordingly, the total area of the catalyst particles in the first layer 61 may be "0", and in this case, the content rate of the catalyst particles in the first layer 61 is also "0".

The fact that the second layer 62 includes more of the catalyst particles than the filler particles can be confirmed by analyzing images of the second layer 62 using the above-described method. The magnifying power of the SEM is selected such that 10 or more (preferably 100 or more) catalyst particles can be observed in one observation field. The content rate of the catalyst particles can be determined by dividing the total area of the catalyst particles by the sum of the total areas of the catalyst particles, the filler particles, and the voids. The content rate of the filler particles can be determined by dividing the total area of the filler particles by the sum of the total areas of the catalyst particles, the filler particles, and the voids.

Note that the second layer 62 does not need to substantially include the filler particles. Accordingly, the total area of the filler particles in the second layer 62 may be "0", and in this case, the content rate of the filler particles in the second layer 62 is also "0".

The porosity of the first layer 61 is preferably larger than the porosity of the second layer 61. In this manner, since the weight of the second layer 62 that acts on the first layer 61 can be more easily absorbed inside the first layer 61, the amount of force that the separation membrane 10 receives from the first layer 61 can be further reduced. As a result, damage to the separation membrane 10 can be further suppressed.

The porositys of the first layer 61 and the second layer 61 can be determined by dividing the total area of the voids determined through the above-described image analysis by the total area.

The value of the porosity of the first layer 61 is not particularly limited, and for example, 30% or more and 60% or less. The value of the porosity of the second layer 62 is not particularly limited, and for example, 20% or more and 50% or less.

### 2. Second embodiment

FIG. 2 is a cross-sectional view of a reactor 2 according to a second embodiment. In FIG. 2, the members that are same as those in FIG. 1 are given the same reference signs as FIG. 1. As shown in FIG. 2, a catalyst 70 included in the reactor 2 according to the second embodiment is different from the catalyst 60 of the reactor 1 according to the first embodiment. Accordingly, in the following description, the difference will be mainly described. Note that in the present embodiment, the non-permeation side flow path 30 does not need to extend in the approximately vertical direction.

The catalyst 70 fills the non-permeation side flow path 30. The catalyst 70 advances (promotes) the conversion reaction in which the raw material gas is converted to the liquid fuel. The catalyst 70 is in direct contact with the separation membrane 10.

The catalyst 70 is constituted by a plurality of catalyst particles and a plurality of filler particles. The configurations of the catalyst particles and the filler particles are as described in the first embodiment. The filler particles are constituted of a material that is softer than the catalyst particles.

The catalyst 70 includes a first portion 71 and a second portion 72.

The first portion 71 is in contact with the separation membrane 10. The first portion 71 corresponds to the circumferentially outward portion of the catalyst 70 in the radial direction of the non-permeation side flow path 30. In the present embodiment, the first portion 71 is formed in a cylindrical shape.

The second portion 72 is separated from the separation membrane 10. The second portion 72 is surrounded by the first portion 71. The second portion 72 is not in contact with the separation membrane 10. The second portion 72 corresponds to the center portion of the catalyst 70 in the radial direction of the non-permeation side flow path 30. In the present embodiment, the second portion 72 is formed in a cylindrical shape.

Note that the first portion 71 does not need to surround the entirety of the second portion 72. The first portion 71 only needs to cover at least a portion of the outer periphery of the second portion 72. In this case, the second portion 72 is disposed in a region of the non-permeation side flow path 30 where the first portion 71 is not disposed.

The thickness of the first portion 71 in the radial direction is not particularly limited, and for example, may be 500 µm or more and 5000 µm or less.

Here, the first portion 71 includes more of the filler particles than the catalyst particles. In other words, in the first portion 71, the content rate of the filler particles is larger than the content rate of the catalyst particles. Thus, the first portion 71 is flexible. As such, when the reactor 1 starts operating, even if a high-temperature material gas flows through the non-permeation side flow path 30 and the entirety of the catalyst 70 thermally expands, the amount of force that the separation membrane 10 receives from the first portion 71 can be reduced. As a result, damage to the separation membrane 10 can be suppressed.

Also, the second portion 72 includes more of the catalyst particles than the filler particles. Since the catalyst particles are constituted of a material that is harder than the filler particles, pulverization of the catalyst particles due to thermal stress can be suppressed.

As described above, according to the catalyst 70 according to the present embodiment, both suppression of damage to the separation membrane 10 and suppression of pulverization of the catalyst 70 can be achieved.

The fact that the first portion 71 includes more of the filler particles than the catalyst particles and the fact that the second portion 72 includes more of the catalyst particles than the filler particles can be confirmed through the image analysis described in the above embodiment.

The porosity of the first portion 71 is preferably larger than the porosity of the second layer 71. Since the flexibility of the first portion 71 can thus be improved, damage to the separation membrane 10 due to thermal expansion of the catalyst 70 can be further suppressed.

The porositys of the first portion 71 and the second layer 71 can be obtained by dividing the total area of the voids determined through the above-described image analysis by the overall total area.

The value of the porosity of the first portion 71 is not particularly limited, and for example, 30% or more and 60% or less. The value of the porosity of the second portion 72 is not particularly limited, and for example, may be 20% or more and 50% or less.

### Modification of embodiment

Although embodiments of the present invention have been described above, the present invention is not limited to the above embodiments, and various modifications can be made without departing from the gist of the invention.

### Modification 1

Although the inner side of the separation membrane 10 is the non-permeation side flow path 30 and the outer side of the separation membrane 10 is the permeation side flow path 40 in the first and second embodiments, a configuration is also possible in which the outer side of the separation membrane 10 is the non-permeation side flow path 30 and the inner side of the separation membrane 10 is the permeation side flow path 40. In this case, it is preferable that the separation membrane 10 is formed surrounding the outer side of the porous support body 20.

### Modification 2

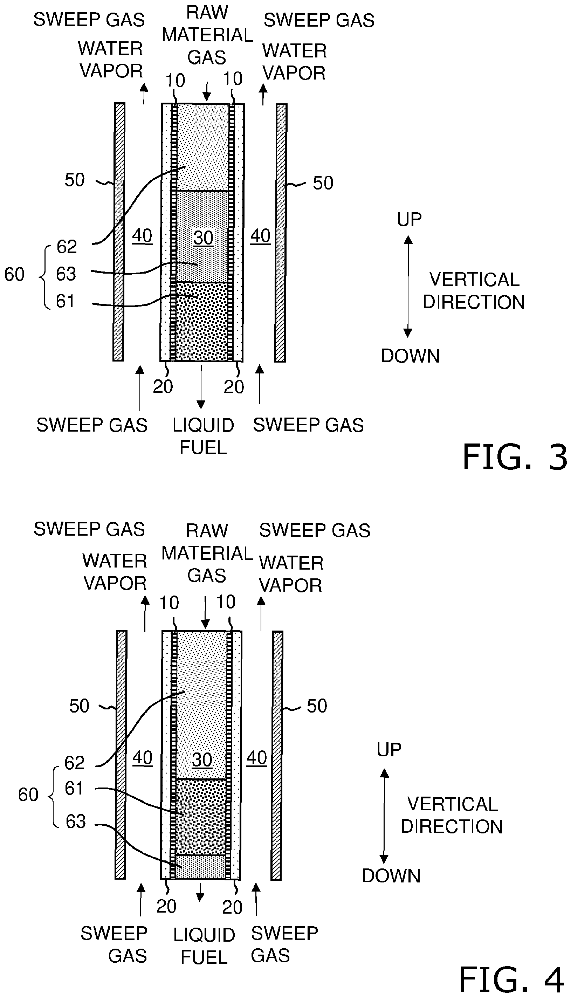

Although the catalyst 60 has a double-layer structure having the first layer 61 and the second layer 62 in the first embodiment, the catalyst 60 may have a structure that includes three or more layers. For example, as shown in FIGS. 3 and 4, the catalyst 60 may have a triple layer structure that includes a third layer 63 in addition to the first layer 61 and the second layer 62.

In FIG. 3, the third layer 63 is disposed between the first layer 61 and the second layer 62 in the approximately vertical direction. It is preferable that the content rate of the filler particles in the third layer 63 is higher than the content rate of the filler particles in the second layer 62. In this manner, the range in which damage to the separation membrane 10 can be suppressed can be larger. The content rate of the filler particles in the third layer 63 may be higher or lower than the content rate of the filler particles in the first layer 61.

In FIG. 4, the third layer 63 is disposed below the first layer 61 in the approximately vertical direction. The third layer 63 includes at least one of the catalyst particles and the filler particles. The third layer 63 may include more of the filler particles than the catalyst particles, or include more of the catalyst particles than the filler particles. In this manner, even in the case where the first layer 61 is not disposed at the lowermost layer, damage to a portion of the separation membrane 10 that is in contact with the first layer 61 can be suppressed. Note that in a case where the third layer 63 includes many catalyst particles, a portion of the separation membrane 10 that is in contact with the third layer 63 may be damaged, and thus it is preferable that the first layer 61 is disposed at the lowermost layer.

### Modification 3

In the first embodiment, in order to suppress damage to the separation membrane 10, the first layer 61 includes more of the filler particles than the catalyst particles. However, instead of or in addition to that, the first layer 61 may include filler particles that are softer than the filler particles included in the second layer 62.

Specifically, of the filler particles included in the catalyst 60, the filler particles disposed in the first layer 61 ("first filler particles" according to the present invention) are softer than the filler particles disposed in the second layer 62 ("second filler particles" according to the present invention). In this manner, the amount of force that the separation membrane 10 receives from the first layer 61 that has thermally expanded can be reduced and the weight of the second layer 62 can be absorbed inside the first layer 61, and thus damage to the separation membrane 10 can be suppressed. "The first filler particles are softer than the second filler particles" means that the hardness of the first filler particles is smaller than the hardness of the second filler particles. The method for measuring the hardness is as described in the first embodiment.

Note that, similarly to Modification 2, in this modification as well, the third layer 63 may be disposed lower than the first layer 61. The third layer 63 includes at least either the catalyst particles or the filler particles. In the case where the third layer 63 includes the filler particles, the filler particles included in the third layer 63 may be softer or harder than the filler particles included in the first layer 61, or have the same hardness as the filler particles included in the first layer 61. Note that if the filler particles included in the third layer 63 are hard, a portion of the separation membrane 10 that is in contact with the third layer 63 may be damaged, and thus it is preferable that the first layer 61 is disposed at the lowermost layer.

### Modification 4

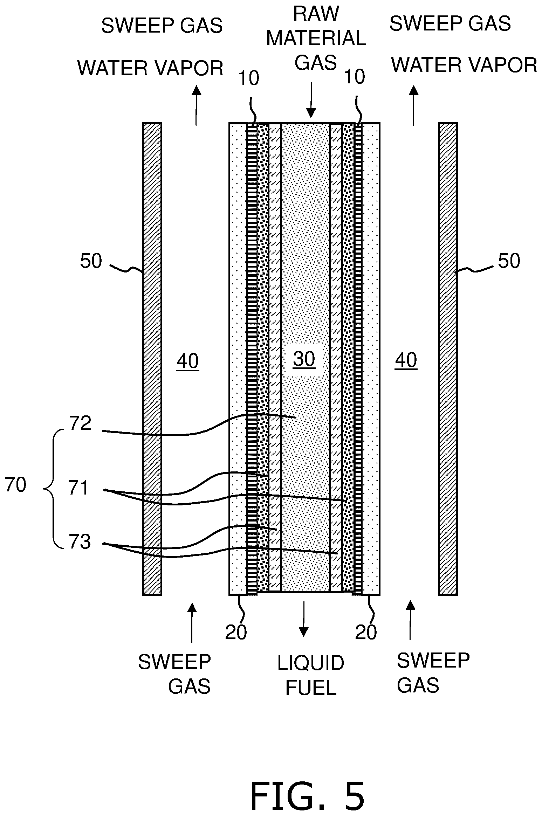

In the second embodiment, the catalyst 70 includes the first portion 71 and the second portion 72. However, another portion may be present between the first portion 71 and the second portion 72. For example, as shown in FIG. 5, the catalyst 70 may further include a third portion 73 in addition to the first portion 71 and the second portion 72.

The third portion 73 is disposed between the first portion 71 and the second portion 72 in the radial direction. The content rate of the filler particles in the third layer 73 is preferably higher than the content rate of the filler particles in the second portion 72. In this manner, the range in which damage to the separation membrane 10 can be suppressed can be larger. The content rate of the filler particles in the third portion 73 may be higher or lower than the content rate of the filler particles in the first portion 71.

### Modification 5

In the second embodiment, in order to suppress damage to the separation membrane 10, the first portion 71 includes more of the filler particles than the catalyst particles. However, instead of or in addition to that, the first portion 71 may include filler particles that are softer than the filler particles included in the second portion 72.

Specifically, of the filler particles included in the catalyst 70, the filler particles disposed in the first portion 71 ("third filler particles" according to the present invention) are softer than the filler particles disposed in the second portion 72 ("fourth filler particles" according to the present invention). In this manner, when the catalyst 70 thermally expands, the amount of force that the separation membrane 10 receives from the first portion 71 can be reduced, and thus damage to the separation membrane 10 can be suppressed. "The third filler particles are softer than the fourth filler particles" means that the hardness of the third filler particles is smaller than the hardness of the fourth filler particles. The method for measuring the hardness is as described in the first embodiment.

### Modification 6

In the second embodiment, in order to suppress damage to the separation membrane 10, the first portion 71 includes more of the filler particles than the catalyst particles, and the second portion 72 includes more of the catalyst particles than the filler particles, but there is no limitation thereto. A configuration is also possible in which the first portion 71 includes more of the catalyst particles than the filler particles, and the second portion 72 includes more of the filler particles than the catalyst particles. In this case as well, since the flexible second portion 72 can absorb thermal expansion force of the entirety of the catalyst 70, the amount of force that the separation membrane 10 receives from the first portion 71 can be reduced. As a result, damage to the separation membrane 10 can be suppressed.

Further, in the case where the first portion 71 includes more of the catalyst particles than the filler particles, and the second portion 72 includes more of the filler particles than the catalyst particles, the second portion 72 may include filler particles that are softer than the filler particles included in the first portion 71. Specifically, of the filler particles included in the catalyst 70, the filler particles disposed in the second portion 72 ("sixth filler particles" according to the present invention) are softer than the filler particles disposed in the first portion 71 ("fifth filler particles" according to the present invention). In this manner, thermal expansion of the entirety of the catalyst 70 can be further absorbed by the second portion 72, and damage to the separation membrane 10 can thus be more suppressed. "The sixth filler particles are softer than the fifth filler particles" means that the hardness of the sixth filler particles is smaller than the hardness of the fifth filler particles. The method for measuring the hardness is as described in the first embodiment.

Further, in the case where the first portion 71 includes more of the catalyst particles than the filler particles, and the second portion 72 includes more of the filler particles than the catalyst particles, the porosity of the second portion may be larger than the porosity of the first portion. In this manner, thermal expansion of the entirety of the catalyst 70 can be further absorbed by the second portion 72, and damage to the separation membrane 10 can thus be further suppressed.

### Modification 7

Although the separation membrane 10 is permeable to water vapor that is a product of the conversion reaction of the raw material gas to the liquid fuel in the above embodiment, the present invention is not limited thereto. The separation membrane 10 may be permeable to the liquid fuel itself, which is a product of the conversion reaction of the raw material gas to the liquid fuel. In this case as well, the reaction equilibrium of the above formula (1) can be shifted to the product side.

Also, when the separation membrane 10 is permeable to the liquid fuel, even when generating the liquid fuel through a reaction in which no water vapor is generated (e.g., 2H₂ + CO ↔ CH₃OH), the reaction equilibrium can be shifted to the product side.

### REFERENCE SIGNS LIST

- 1: Reactor
- 10: Separation membrane
- 20: Porous support body
- 30: Non-permeation side flow path
- 30a: Upper end opening
- 30b: Lower end opening
- 40: Permeation side flow path
- 40a: Lower end opening
- 40b: Upper end opening
- 50: Outer tube
- 60: Catalyst
- 61: First layer
- 62: Second layer
- 63: Third layer
- 70: Catalyst
- 71: First portion
- 72: Second portion
- 73: Third portion

## Claims

1. A reactor comprising:
a separation membrane permeable to a product of a conversion reaction in which a raw material gas containing at least hydrogen and carbon oxide is converted to a liquid fuel;
a non-permeation side flow path provided on a non-permeation side of the separation membrane, the raw material gas flowing through the non-permeation side flow path; and
a catalyst provided in the non-permeation side flow path and configured to promote the conversion reaction, wherein
the catalyst includes catalyst particles each constituted by a carrier and a supported catalytic component, and filler particles softer than the catalyst particles.

2. The reactor according to claim 1, wherein
the non-permeation side flow path extends in an approximately vertical direction,
the catalyst has a first layer and a second layer disposed higher than the first layer,
the first layer includes more of the filler particles than the catalyst particles, and
the second layer includes more of the catalyst particles than the filler particles.

3. The reactor according to claim 1 or 2, wherein
the non-permeation side flow path extends in an approximately vertical direction,
the catalyst has a first layer and a second layer disposed higher than the first layer,
the filler particles include first filler particles disposed in the first layer and second filler particles disposed in the second layer, and
the first filler particles are softer than the second filler particles.

4. The reactor according to claim 3, wherein
a porosity of the first layer is larger than a porosity of the second layer.

5. The reactor according to claim 2, wherein
the first layer is located at a lowermost layer of the catalyst.

6. The reactor according to claim 1, wherein
the catalyst has a first portion in contact with the separation membrane, and a second portion separated from the separation membrane,
the first portion includes more of the filler particles than the catalyst particles, and
the second portion includes more of the catalyst particles than the filler particles.

7. The reactor according to claim 1 or 6, wherein
the catalyst has a first portion in contact with the separation membrane, and a second portion separated from the separation membrane,
the filler particles include third filler particles disposed in the first portion and fourth filler particles disposed in the second portion, and
the third filler particles are softer than the fourth filler particles.

8. The reactor according to claim 6, wherein
a porosity of the first portion is larger than a porosity of the second portion.

9. The reactor according to claim 1, wherein
the catalyst has a first portion in contact with the separation membrane, and a second portion separated from the separation membrane, and
the first portion includes more of the catalyst particles than the filler particles, and
the second portion includes more of the filler particles than the catalyst particles.

10. The reactor according to claim 1 or 9, wherein
the catalyst has a first portion in contact with the separation membrane, and a second portion spaced apart from the separation membrane,
the filler particles include fifth filler particles disposed in the first portion and sixth filler particles disposed in the second portion, and
the sixth filler particles are softer than the fifth filler particles.

11. The reactor according to claim 9, wherein
a porosity of the second portion is larger than a porosity of the third portion.

12. The reactor according to claim 1, wherein
the filler particles are each constituted by a carrier and a supported catalytic component.
